(19) Europäisches Patentamt
European Patent Office
Office européen des brevets

(11) Publication number: 0 057 353
B1

(12) EUROPEAN PATENT SPECIFICATION

(45) Date of publication of patent specification: 22.01.86

(51) Int. Cl.[4]: A 61 K 7/06

(21) Application number: 82100202.9

(22) Date of filing: 13.01.82

# (54) Hair rinse composition.

(30) Priority: 27.01.81 JP 10471/81

(43) Date of publication of application:
11.08.82 Bulletin 82/32

(45) Publication of the grant of the patent:
22.01.86 Bulletin 86/04

(84) Designated Contracting States:
AT BE CH DE FR IT LI NL

(56) References cited:
EP-A-0 052 436
EP-A-0 052 441
EP-A-0 052 943
EP-A-0 053 448
US-A-3 961 634

(73) Proprietor: KAO SOAP COMPANY LIMITED
1-1 Nihonbashi Kayaba-cho Chuo-ku
Tokyo (JP)

(72) Inventor: Abe, Yoshiaki
6-35-3, Higashinippori
Arakawa-ku Tokyo (JP)
Inventor: Tsushima, Rikio
4-21-302, Akiba-cho
Wakayama-shi Wakayama-ken (JP)

(74) Representative: Wächtershäuser, Günter, Dr.
Tal 29
D-8000 München 2 (DE)

EP 0 057 353 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to hair rinse compositions which comprise derivatives of keratin material therein and show an excellent hair conditioning effect. Hair rinse compositions of this type are also described in an earlier European patent application 0052441 of the applicant.

DESCRIPTION OF THE PRIOR ART

On washing hair with shampoos mainly composed of anionic surface active agents to remove dirt from the hair, it will be found that the oil component protecting the hair surface as well as dirt is removed. The removal of the oil component from the hair surface results in a loss of softness of the hair and thus the hair becomes lusterless and hard to comb, producing a tendency towards the damge of the hair, split-ends or broken hairs.

In order to prevent these troubles and condition the hair to impart softness, smoothness and wetness to the touch and improving combing ease, hair rinses have conventionally been used.

Known hair rinses are fundamentally comprise of quaternary ammonium salts which are a cationic surface active agent serving to impart softness and smoothness to hair, and oil components such as liquid paraffin, higher alcohols, and the like for supplementing the oil component in the hair to form an oil film on the hair surface so that the hair appears glossy. Damage due to the frictional contact with brush, comb and the like is reduced, and split ends or broken hairs are prevented.

However, quaternary ammonium salts are not capable of stably emulsifying and dispersing oil components in amounts sufficient to produce the effects as mentioned above and thus hair rinses using such salts become unstable. Where nonionic surface active agents are added in order to overcome the above drawback, there is involved the disadvantage that the inherent rinsing effect is lowered. Accordingly, there have been proposed hair rinses in which there are incorporated instead of the oil component anionic surface active agents, anionic polymer compounds, cationic polymer compounds, and hydrolysates of collagen. However, these rinses are not satisfactory.

Further, it is known from US—A—3967634 that a hydrolysate of keratin renders a composition for bleaching hair less damaging to the hair.

It is accordingly an object of the present invention to provide hair rinse compositions which show excellent hair conditioning effects.

It is another object of the invention to provide hair rinse compositions which comprise derivatives of keratin material and which condition hair better than known hair rinses.

It is a further object of the invention to provide hair rinse compositions which can impart good feeling of the hair to the touch.

According to the present invention, there is provided a hair rinse composition which comprises, in liquid medium, 0.01—10 wt% of at least one derivative of keratin material selected from salts with bases of products obtained by oxidation of keratin material and salts with bases of derivatives of the mercapto groups of products obtained by reduction of keratin material, wherein the derivative of the mercapto group is as defined in claim 1. Hair rinse compositions are excluded from the claims of the present invention.

The keratin derivatives to be used in the present invention can be prepared by oxidising keratin material by oxidation and converting the product into a salt with a base, and reducing a keratin material, chemically modifying the mercapto groups of the product to obtain a derivative thereof, and converting the derivative into a salt with a base.

The starting keratin materials include, for example, animal hair, human hair, feathers, nails, horns, hooves and scale, among which wool, human hair and feathers are preferably used. These keratin materials may be subjected to oxidation or reduction directly but if necessary, they may be cut or reduced into pieces of a suitable size or subjected to pretreatments such as washing and defatting.

The derivatives of the keratin material are prepared by any of the following methods.

(1) Oxidation Reaction

The oxidation of keratin material is feasible by any of methods known per se (N.H. Leon; Textile Progress, Vol. 7, Page 1 (1975)). Oxidizing agents are preferably either organic or inorganic oxidising agents that act electrophilically on the disulfide bonds (S—S bonds) in the keratin structure. Examples of the oxidizing agents include organic peracids, inorganic peroxo acids or their salts, permanganic acid or its salts, chromic acid or related compounds, halogens, peroxides and oxyacid or their salts, among which peracetic acid, performic acid and perbenzoic acid are most preferred.

The oxidation reaction is conducted in a liquid medium using the oxidizing agent in excess with respect to the disulfide bonds in keratin material generally in amounts of two equivalents or more, preferably 4—10 equivalents, per disulfide bond. The reaction is feasible under acidic or alkaline conditions and is preferably conducted under acidic and particularly weakly acidic conditions. The reaction temperature and pressure depend on the oxidizing agent and keratin material used and thus are not critical. In general, room temperature is sufficient but if necessary, heat may be applied. Atmospheric pressure is sufficient but the reaction may be carried out under reduced pressure or under pressure.

Upon oxidation the disulfide bonds of the keratin material are converted into sulfo groups.

(2) Reduction Reaction and Chemical Modification Reaction

Reducing agents employed for reducing keratin materials are preferably organic or inorganic reducing agents that act nucleophilically on the disulfide bonds to convert the disulfide bonds in the keratin structure into mercapto groups (—SH). Examples of the reducing agents include organic reducing agents such as 2-mercaptoethanol, thioglycollic acid, benzylmercaptan, 1,4-dithiothreitol and tributylphosphine, and inorganic reducing agents such as sodium hydrogensulfite, sulfides such as sodium hydrosulfide and metallic hydrides such as lithium aluminium hydride.

The amount of the reducing agent is usually in the range of 2—10 equivalents with respect to the disulfide bonds in keratin material. The pH of the reaction system is in the range of 2—12, preferably 6—11. Outside this range, undesired hydrolysis takes place. Room temperature is sufficient but heat may be applied to shorten the reaction time which is ordinarily in the range of 2—3 hours or more. Since it is necessary that the mercapto groups produced by the reduction should not be substantially oxidized, the reduction operation should be preferably carried out in an atmosphere of inert gas to give good results.

The mercapto groups obtained by the reduction of the keratin material is then chemically modified to obtain a derivative thereof. The derivatives of the mercapto groups are:

$-SCH_2COOH$, $-SCH_2CH_2COOH$, 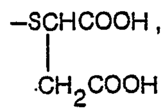,

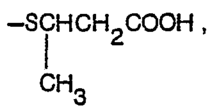, 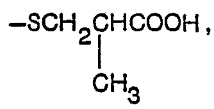,

$-SSO_3H$, $-SCH_2CH_2SO_3H$, 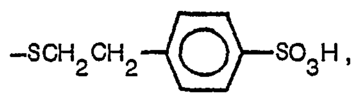,

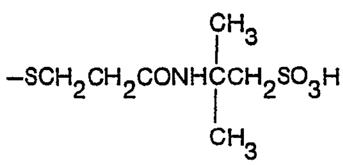 and $-SCH_2CH_2SO_2CH_2COOH$,

among which $-SCH_2COOH$ and 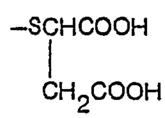 are preferred.

The chemical modification of the mercapto group can be carried out by any procedures known per se, for example, on the basis of procedures described in N. H. Loen; Textile Progress, Vol. 7, page 1 :1975), "Yuuki Ioo Kagobutsu (Organic Sulfur Compounds)" written by Shigeru Ookyo and published by Kagaku Dojin (1968), and "Kobunshi Jikkengaku Koza" written by Masami Oku, Vol. 12, Kyoritsu Shuppan (1975). Typical methods are described below.

(1) Method utilizing the nucleophilic substitution reaction of SH group

$$K - SH + R - L \rightarrow K - S - R + HL$$

(in which K represents a residue of the keratin compound, R represents a chemically modifying group to be introduced, and L represents a releasing atom or group such as a halogen atom or an acid residue).

Compounds reacting by this method include, for example, halogen compounds such as iodoacetic acid, bromoacetic acid and chloroacetic acid.

(2) Method utilizing the nucleophilic addition reaction of SH group with a carbon-carbon double bond

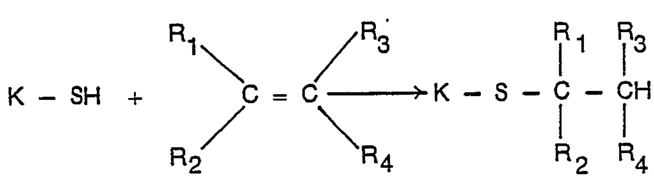

(in which at least one of $R_1$, $R_2$, $R_3$ and $R_4$ represents a carboxyl group or sulfo group, the other represent an

alkyl group or a hydrogen atom, and K has the same meaning as defined hereinbefore).

Compounds reacting by this method include, for example, acrylic acid, methacrylic acid, crotonic acid, maleic acid, fumaric acid, vinylcarboxymethylsulfonic, vinylsulfonic acid, p-styrenesulfonic acid and 2-acrylamido-2-methylpropanesulfonic acid.

(3) Method using a substitution reaction between SH group and sulfite compound

$$K - SH + NaHSO_3 \rightarrow K - S - SO_3H$$

$$K - SH + Na_2SO_3 \xrightarrow[\text{air}]{} K - S - SO_3H$$

(in which K has the same meaning as defined hereinbefore).

Salt, of the oxidation products and the derivatives of the reduced keratin material include salts with organic alkalis such as sodium and potassium, ammonium salts, salts with organic bases such as ethanolamine, diethanolamine, triethanolamine, 2-amino-2-methylpropanol, triisopropanolamine, glycine, histidine and arginine. These salts may be prepared separately and incorporated in the hair rinse composition. Alternatively, oxidation products of the keratin material or the derivatives of the reduced keratin material and base, may be separately added to the hair rinse composition and the salts formed in situ. In the latter case, useful bases include, for example, sodium hydroxide, potassium hydroxide, aqueous ammonia, ethanolamine, diethanolamine, triethanolamine, 2-amino-2-methylpropanol, triisopropanolamine, glycine, arginine and histidine. Preferably, these bases are added in an amount of 0.1—8 equivalents of the carboxyl and sulfo groups in the oxidation product of the keratin material the derivative of the reduced keratin material.

The hair rinse composition according to the invention can be prepared by dissolving or dispersing 0.01—10 wt% (hereinafter referred to simply as %), preferably 0.1—5%, of one or more of derivatives of keratin material as defined above in a suitable solvent or liquid medium such as water, ethanol, glycerine, ethylene glycol, propylene glycol, 1,3-propanediol, isopropanol or polyethylene glycol.

Less than 0.01% of derivatives of the keratin material are unfavorable since a satisfactory effect is not produced, whereas larger amounts than 10% are not favorable since the hair becomes undesirably sticky under high humidity conditions.

Aside from the essential component of the keratin derivatives known ingredients which are employed in ordinary hair rinses may be added to the hair rinse compositions of the present invention. It is preferable to incorporate a surface active agent selected from anionic surface active agents, nonionic surface active agents and amphoteric surface active agents as an auxiliary component.

Examples of these surface active agents are as follows.

1) Anionic Surface Active Agents

* Linear or branched alkylbenzenesulfonates having alkyl groups having an average of 10—16 carbon atoms.

* Ethoxylated alkyl or alkenyl sulfates having a linear or branched alkyl or alkenyl group having an average of 8—20 carbon atoms and having an average of 0.5—8 moles of ethylene oxide added.

* Alkyl or alkenylsulfates having an average of 10—20 carbon atoms.

* Olefinsulfonates having an average of 10—20 carbon atoms per molecule.

* Alkanesulfonates having an average of 10—20 carbon atoms per molecule.

* Saturated or unsaturated fatty acid salts having an average of 10—20 carbon atoms per molecule.

* Alkyl or alkenyl ethoxycarbonates having an alkyl or alkenyl group having an average of 10—20 (preferably 12—16) carbon atoms and having an average of 0.5—8 moles of ethylene oxide added per molecule.

* α-sulfofatty acid salts or esters of the following formula

$$\begin{array}{l} R_5CHCO_2Y \\ \quad | \\ \quad SO_3M \end{array}$$

(in which Y represents an alkyl group having 1—3 carbon atoms or a counter ion, M is a counter ion, and $R_5$ represents an alkyl or alkenyl group having 10—20 (preferably 12—16) carbon atoms).

The counter ions of the anionic surface active agent include alkali metal ions such as sodium and potassium, alkaline earth metal ions such as calcium and magnesium, ammonium ion, ions of alkanolamines having 1—3 hydroxyalkyl groups having 2—3 carbon atoms (such as, for example, monoethanolamine, diethanolamine, triethanolamine and triisopropanolamine).

2) Nonionic Surface Active Agents

* Polyoxyethylene alkyl or alkenyl ethers having a primary or secondary alkyl or alkenyl group having an average of 8—20 carbon atoms and having 3—12 moles of ethylene oxide added thereto.

* Polyoxyethylene alkylphenyl ethers having alkyl groups having an average of 8—12 carbon atoms and having 3—12 moles of ethylene oxide added thereto.

* Higher fatty acid alkanolamides of the following formula of their alkylene oxide adducts

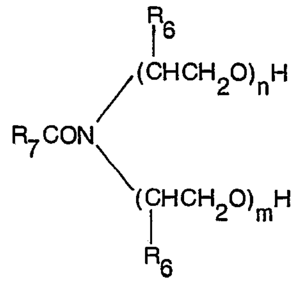

(in which $R_6$ represents H or $CH_3$, $R_7$ represents an alkyl or alkenyl group having 10—20 carbon atoms, n is an integer of 1—3, and m is an integer of 0—3).

3) Amphoteric Surface Active Agents

a. Alkylamine oxides of the following formulas

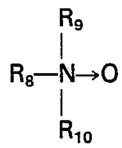

(in which $R_8$ represents an alkyl or alkenyl group having 10—20 carbon atoms, and $R_9$ and $R_{10}$ are independently an alkyl group having 1—3 carbon atoms).

It is preferable that, in the above formula, $R_8$ contains 12—16 carbon atoms and both $R_9$ and $R_{10}$ are each a methyl group.

b.

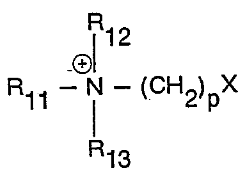

(in which $R_{11}$ represents an alkyl or alkenyl group having 10—20 carbon atoms, $R_{12}$ and $R_{13}$ independently represent an alkyl group having 1—4 carbon atoms, p is an integer of 1—3, and X represents a $—COO^-$ or $—SO_3^-$ group).

Preferably, in the above formula, $R_{11}$ is the group having 12—16 carbon atoms, $R_{12}$ and $R_{13}$ independently a methyl group, and p is a value of 3.

c. Imidazoline compounds of the following formula

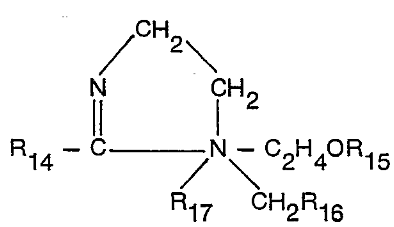

(in which $R_{14}$ represents a fatty acyl group having an average of 10—20 carbon atoms, $R_{15}$ represents hydrogen, Na, or $CH_2COOMe$ (Me: H, Na, or an organic base), $R_{16}$ represents COOMe, $CH_2COOMe$ or

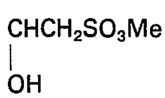

(Me has the same meaning as defined above), and $R_{17}$ represents a hydroxyl group, an acidic salt, or an anionic surface active sulfate or sulfatized product).

Preferably, in the above formula, $R_{14}$ represents a fatty acyl group having 12—16 carbon atoms.

Among these surface active agents, the anionic surface active agents and particularly ethoxylated alkyl

sulfates having a linear or branched alkyl group having 12—16 carbon atoms and having 1—4 moles of ethylene oxide added per molecule thereof or linear or branched alkylsulfates having an average 12—16 carbon atoms are preferred.

Good results are obtained when these surface active agents are added in an amount of 0.01—5%, preferably 1—2%, based on the hair rinse composition.

Moreover, there may be added to the hair rinse composition of the invention a variety of ingredients which include: oils including hydrocarbons such as liquid paraffin, Vaseline® and solid paraffin, esters such as isopropylmyristate, lanolin derivatives such as lanolin, refined lanolin and lanolin fatty acids, silicone derivatives such as dimethylpolysiloxane, methylphenylpolysiloxane and organo-modified polysiloxanes, and polyethylene glycol, polypropylene glycol or its polymer, polyoxyalkylene alkyl ethers and polyoxyalkylene alkyl ether phosphotes; polymeric materials such as hydroxyethyl cellulose, hydroxypropylmethyl cellulose, hydroxypropyl cellulose, methyl cellulose, cationized cellulose and cationized polymers; and germicides, preservatives, perfumes and dyes.

The present invention is particularly described by way of Reference Examples and Examples.

Reference Example 1

Preparation of derivatives by oxidation of keratin materials:

(a) Ten grams of wool fibers were immersed in 700 g of a 8% aqueous peracetic acid solution at room temperature for 1 day. The resulting oxidized wool fibers were filtered, washed with water and immersed in 700 g of a 0.1N aqueous ammonia at room temperature for 1 day, about 90% of the wool fibers dissolved in the aqueous ammonia. About 1 g of insoluble material was removed by filtration and the pH of the ammoniacal solution of keratose (oxidized product of woll keratin) was adjusted to 4.0 with 2N hydrochloric acid, whereupon α-keratose precipitated. This precipitate was filtered, washed with acetone and dried to yield 5.4 of α-keratose.

(b) Wool fibers were heated under pressure in an autoclave by the use of saturated steam at 6 kg/cm$^2$ for 6 minutes and were abruptly released into the atmosphere to yield a porous puffed product. Ten grams of the puffed product, which had been reduced to pieces, were immersed in 250 g of formic acid and 50 g of a 30% aqueous hydrogen peroxide solution in a 500 ml three neck flask at room temperature for 1 day, whereupon no powder was found in the solution but foam-like masses floated in the upper layer. This reaction mixture was filtered and the filtrate was poured into 1.5 litres of water, followed by adding hydrochloric acid to adjust the pH to 4. The resulting precipitate was collected by filtration and washed with 500 ml of water to yield 4.5 g of α-keratose. To the insoluble matter from which the reaction product had been removed were added 350 ml of water and then the pH was adjusted to 11 by adding aqueous ammonia. The mixture was allowed to stand at room temperature for 1 day. The mixture was filtered and hydrochloric acid was added to the filtrate to adjust the pH to 4. The resulting precipitate was collected by filtration to yield 0.7 g of α-keratose. The insoluble material (1.4 g) was primarily β-keratose.

Reference Example 2

Preparation of derivatives by reduction of keratin materials:

(a) Ten grams of wool fibers were immersed in 600 ml of an aqueous solution of 8M urea and 0.01M Tris buffer, to which was added 6 ml of 2-mercaptoethanol, followed by adjusting the pH to 10 by means of a 5N potassium hydroxide aqueous solution. The reduction reaction was carried out in a stream of nitrogen at room temperature. About 3 hours after commencement of the reaction, 85% of the wool had dissolved in the reaction solution. While controlling the pH of the reaction mixture to a pH not lower than 7 by using an aqueous 5N potassium hydroxide solution, 16.5 g of iodoacetic acid was gradually added and the pH of the mixture was finally adjusted to 8.5. The carboxymethylation reaction was carried out at room temperature for 2 hours. The reaction solution was filtered to remove insoluble material from the solution and the resultant filtrate was charged into a cellulose tube wherein it was dialyzed against ion-exchanged water to remove low molecular weight impurities including urea. As the urea was dialyzed, the content in the cellulose tube became cloudy since HGT (component with high contents of glycine and tyrosine), a water-insoluble material, precipitated. After completion of the dialysis, the HGT was removed by centrifugal separation and S-carboxymethyl keratin (SCMKA) was obtained from the neutral transparent aqueous solution of SCMKA by isoelectric precipitation. That is, 1N hydrochloric acid was added to the mixture to adjust its pH to 4.4 to precipitate the SCKMA. This precipitate was filtered, washed with ethanol and dried to yield 4.2 g of SCMKA.

(b) The procedure of Reference Example 2 (a) was repeated except that feathers, which were heated for 6 minutes in an autoclave by means of superheated steam at 6 kg/cm$^2$ and 240°C and then abruptly released into the atmosphere to yield a porous puffed product, more used instead of wool fibers and 1.75 g of maleic acid was used instead of iodoacetic acid, thereby yielding 5.3 g of S-(1,2-dicarboxyethyl)-keratin.

(c) The procedure of Reference Example 2 (a) was repeated using powdered horse's hooves instead of wool fibers and 11 g of acrylic acid instead of iodoacetic acid, thereby yielding 4.2 g of S-(2-carboxyethyl)-keratin.

(d) The procedure of Reference Example 2 (a) was repeated using 28 g of p-styrenesulfonic acid instead of iodoacetic acid, thereby yielding 4.2 g of S-(p-sulfophenyl)-keratin.

(e) Eight grams of wool fibers were dispersed in 300 ml of n-propanol and 300 ml of a 0.1N Tris buffer

solution. After replacing the air with nitrogen, 3.2 ml of tri-n-butylphosphine was added, followed by agitating at room temperature for 24 hours. The solution was filtered and to the resulting insoluble material was added 400 ml of water, 9.28 g of maleic acid and about 30 ml of 5N potassium hydroxide to adjust the pH 8.0, followed by agitating at room temperature for 6 hours. About 20 ml of aqueous ammonia was adding to the reaction mixture to adjust the pH to 11.5, after which it was agitated for 18 hours at room temperature. The reaction mixture was filtered to remove insoluble material therefrom and the resulting filtrate was placed in a cellulose tube in which it was dialyzed against ion-exchanged water to remove low molecular weight impurities. After completion of the dialysis, the insoluble material in the cellulose tube were removed by centrifugal separation and the pH of the resulting neutral transparent aqueous solution was adjusted to 4.4 by the addition of about 5.5 ml of 1N hydrochloric acid and the resulting precipitate was collected by filtration, followed by washing with ethanol and drying to yield 3.9 g of S-(1,2-dicarboxyethyl)-keratin.

(f) The procedure of Reference Example 2 (e) was repeated except that a powdered porous puffed product of wool obtained by heating wool in an autoclave by means of saturated steam at 6 kg/cm$^2$ for 6 minutes was used instead of wool fibers and 16.5 g of 2-acrylamido-2-methylpropanesulfonic acid was used instead of maleic acid, thereby yielding 4.5 g of 5-[2-(1,1-dimethyl-2-sulfoethyl-carbamoyl)ethyl]-keratin.

## Example

Hair with a weight of 10 g and a length of 10 cm was treated with the following hair rinse compositions and completely dried by a dryer to compare its softness, gloss and resilience (tensity and firmness of hair) with non-treated hair. The comparison was made by an expert panel of ten female members according to the following evaluation standard. Table 1 shows average values of the evaluation.

Formulation:

| | |
|---|---|
| Derivative of keratin (in the case of compositions of invention) | 1.0(%) |
| Surface active agent or oil | 1.0 |
| Water | balance |
| Caustic soda | suitable amount (pH 7.0) |

Evaluation Standard:

| Evaluation Point | Softness<br>As compared with non-treated hair | Gloss<br>As compared with non-treated hair | Resilience<br>As compared with non-treated hair |
|---|---|---|---|
| 5 | much better | much better | much better |
| 4 | better | better | better |
| 3 | equal | equal | equal |
| 2 | slightly poorer | slightly poorer | slightly poorer |
| 1 | poorer | poorer | poorer |

Results:

TABLE 1

| | Derivative of Keratin | Surface Active Agent or Oil | Softness | Gloss | Resilience |
|---|---|---|---|---|---|
| 1 | – | – | 3.0 | 3.0 | 3.0 |
| 2 | – | cetyltrimethyl-ammonium chloride | 4.0 | 3.5 | 0.5 |
| 3 | – | purified lanolin | 3.6 | 3.5 | 1.8 |
| 4 | – | N-laurylbetaine | 2.5 | 3.0 | 3.1 |
| 5 | – | – | 2.7 | 3.1 | 3.3 |
| 6 | – | myristylamine oxide | 2.5 | 3.0 | 2.1 |
| 7 | – | ammonium laurylsulfate | 3.5 | 3.5 | 2.9 |
| 8 | – | polyoxyethylene nonyl-phenyl-ether | 3.3 | 3.1 | 2.5 |
| 9 | product of Reference Example 1(a) | – | 3.9 | 4.2 | 4.5 |
| 10 | product of Reference Example 1(b) | – | 3.7 | 4.1 | 4.2 |
| 11 | product of Reference Example 2(a) | – | 4.0 | 3.9 | 4.0 |
| 12 | product of Reference Example 2(c) | – | 3.9 | 4.2 | 3.9 |
| 13 | product of Reference Example 1(a) | N-laurylbetaine | 4.0 | 3.9 | 4.2 |
| 14 | ,, | laurylimidazoline | 4.1 | 3.8 | 4.3 |
| 15 | ,, | myristylamine oxide | 4.0 | 4.0 | 3.9 |
| 16 | ,, | ammonium laurylsulfate | 4.2 | 4.5 | 3.6 |
| 17 | ,, | polyoxyethylene nonylphenyl ether | 4.0 | 3.6 | 4.0 |
| 18 | ,, | purified lanolin | 4.8 | 4.5 | 4.4 |
| 19 | product of Reference Example 2(a) | N-laurylbetaine | 4.5 | 3.5 | 4.0 |
| 20 | ,, | laurylimidazoline | 4.1 | 3.9 | 3.8 |
| 21 | ,, | myristylamine oxide | 3.8 | 4.1 | 3.9 |
| 22 | ,, | ammonium laurylsulfate | 4.2 | 4.2 | 4.0 |
| 23 | ,, | polyoxyethylene nonylphenyl ether | 4.0 | 3.7 | 3.7 |
| 24 | ,, | purified lanolin | 4.6 | 3.6 | 3.9 |

**Claims for the Contracting States: BE CH DE FR IT LI NL**

1. A hair rinse composition comprising, in liquid medium, 0.1 to 10 wt.-% of at least one derivative of keratin material selected from salts with bases of products obtained by oxidation of keratin material and salts with bases of derivatives of the mercapto groups of products obtained by the reduction of keratin material, wherein the derivative of the mercapto group is selected from

$-SCH_2CH_2COOH$, 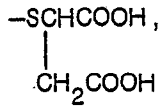 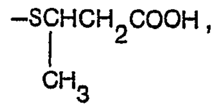

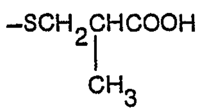 $-SCH_2COOH$, $-SSO_3H$, $-SCH_2CH_2SO_3H$, 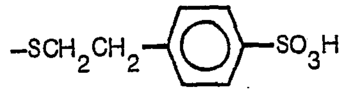

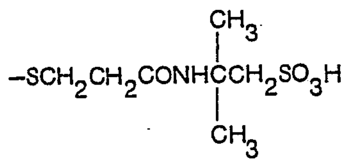 and $-SCH_2CH_2SO_2CH_2COOH$;

with the exception of hair rinse compositions containing a cationic surface active agent.

2. A hair rinse composition according to Claim 1 comprising 0.1—5 wt% of the composition of at least one derivative of keratin material.

3. A hair rinse composition according to Claim 1, further comprising 0.01—5 wt% of at least one surface active agent selected from anionic surface active agents, nonionic surface active agents and amphoteric surface active agents.

4. A hair rinse composition according to Claim 3 comprising 1—2 wt% of the composition of at least one surface active agent.

5. A hair rinse composition according to Claim 3 or 4, wherein at least one of the surface active agents is an ethoxylated alkyl sulfate having a linear or branched alkyl group having 12—16 carbon atoms and having 1—4 moles of ethylene oxide per molecule thereof or a linear or branched alkylsulfate having an average of 12—16 carbon atoms.

**Claims for the contracting state: AT**

1. A process for producing a hair rinse composition comprising an addition of 0.1 to 10 wt.% of at least one derivative of keratin material selected from salts with bases of products obtained by oxidation of keratin material and salts with bases of derivatives of the mercapto groups of products obtained by the reduction of keratin material, wherein the derivative of the mercapto group is selected from

$-SCH_2CH_2COOH$, 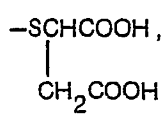 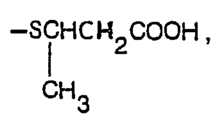

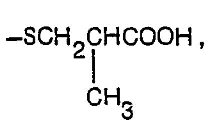 $-SCH_2COOH$, $-SSO_3H$, $-SCH_2CH_2SO_3H$, 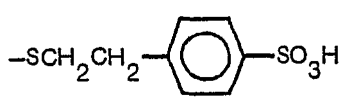

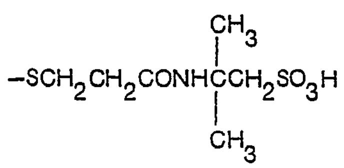 and $-SCH_2CH_2SO_2CH_2COOH$;

to a liquid medium; with the exception of hair rinse compositions containing a cationic surface active agent.

2. The process according to claim 1, comprising an addition of 0.1 to 5 wt.% of the composition of at

least one derivative of keratin material.

3. The process according to claim 1, further comprising an addition of 0.01 to 5 wt.% of at least one surface active agent selected from anionic surface agents, nonionic surface active agents and amphoteric surface active agents.

4. The process according to claim 3 comprising an addition of 1 to 2 wt.% of the composition of at least one surface active agent.

5. The process according to claim 3 or 4, wherein at least one of the surface active agents is an ethoxylated alkyl sulfate having a linear or branched alkyl group having 12 to 16 carbon atoms and having 1 to 4 moles of ethylene oxide per molecule thereof or a linear or branched alkyl sulfate having an average of 12 to 16 carbon atoms.

6. A hair rinse composition comprising, in liquid medium, 0.1 to 10 wt.% of at least one derivative of keratin material selected from salts with bases of products obtained by oxidation of keratin material and salts with bases of derivatives of the mercapto groups of products obtained by the reduction of keratin material, wherein the derivative of the mercapto group is selected from

$-SCH_2CH_2COOH$, 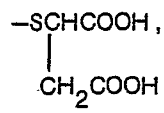, 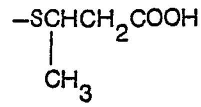,

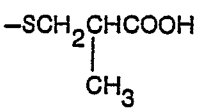, $-SCH_2COOH$, $-SSO_3H$, $-SCH_2CH_2SO_3H$, 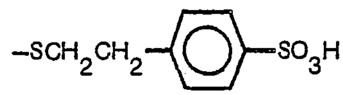,

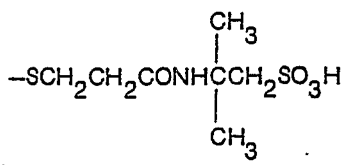 and $-SCH_2CH_2SO_2CH_2COOH$;

with the exception of hair rinse compositions containing a cationic surface active agent.

7. A hair rinse composition according to claim 6, comprising 0.1 to 5 wt.% of the composition of at least one derivative of keratin material.

8. A hair rinse composition according to claim 6, further comprising 0.01 to 5 wt.% of at least one surface active agent selected from anionic surface active agents, nonionic surface active agents and amphoteric surface active agents.

9. A hair rinse composition according to claim 8, comprising 1 to 2 wt.% of the composition of at least one surface active agent.

10. A hair rinse composition according to claim 8 or 9, wherein at least one of the surface active agents is an ethoxylated alkyl sulfate having a linear or branched alkyl group having 12 to 16 carbon atoms and having 1 to 4 moles of ethylene oxide per molecule thereof or a linear or branched alkylsulfate having an average of 12 to 16 carbon atoms.

**Patentansprüche für die Vertragsstaaten: BE CH DE FR IT LI NL**

1. Haarspülmittel, umfassend in flüssigem Medium 0,1 bis 10 Gew.% mindestens eines Derivats von Keratinmaterial, ausgewählt aus Basensalzen von Produkten, die durch Oxidation von Keratinmaterial erhalten wurden, und Basensalzen von Derivaten der Mercaptogruppen von Produkten, die durch Reduktion von Keratinmaterial erhalten wurden, wobei das Derivat der Mercaptogruppe ausgewählt ist aus

$-SCH_2CH_2COOH$, 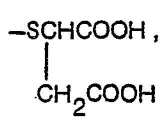, 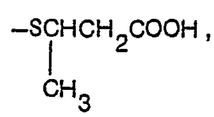, 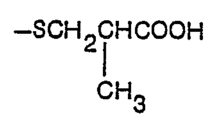,

$-SCH_2COOH$, $-SSO_3H$, $-SCH_2CH_2SO_3H$, 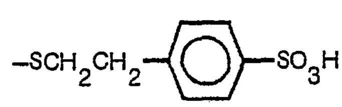,

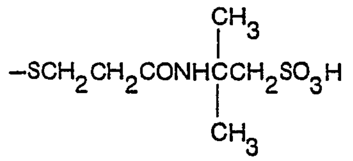 und $-SCH_2CH_2SO_2CH_2COOH$;

mit Ausnahme von Haarspülmitteln, welche ein kationisches oberflächenaktives Mittel enthalten.

2. Haarspülmittel nach Anspruch 1, umfassend mindestens ein Derivat von Keratinmaterial in einer Menge von 0,1 bis 5 Gew.% des Mittels.

3. Haarspülmittel nach Anspruch 1, ferner umfassend mindestens ein oberflächenaktives Mittel in einer Menge von 0,01 bis 5 Gew.%, ausgewählt aus anionischen oberflächenaktiven Mitteln, nicht ionischen oberflächenaktiven Mitteln und amphoteren oberflächenaktiven Mitteln.

4. Haarspülmittel nach Anspruch 3, wobei mindestens ein oberflächenaktives Mittel in einer Menge von 1 bis 2 Gew.% des Haarspülmittels enthalten sind.

5. Haarspülmittel nach Anspruch 3 oder 4, wobei mindestens eines der oberflächenaktiven Mittel ein ethoxyliertes Alkylsulfat mit einer linearen oder verzweigten Alkylgruppe mit 12 bis 16 Kohlenstoffatomen und mit 1 bis 4 Mol Ethylenoxyd pro Molekül desselben ist, oder ein lineares oder verzweigtes Alkylsulfat mit durchschnittlich 12 bis 16 Kohlenstoffatomen.

**Patentansprüche für den Vertragsstaat: AT**

1. Verfahren zur Herstellung eines Haarspülmittels, dadurch gekennzeichnet, daß man einem flüssigen Medium 0,1 bis 10 Gew.% mindestens eines Derivats von Keratinmaterial zusetzt, ausgewählt aus Basensalzen von Produkten, die durch Oxidation von Keratinmaterial erhalten wurden, und Basensalzen von Derivaten der Mercaptogruppen von Produkten, die durch die Reduktion von Keratinmaterial erhalten wurden, wobei das Derivat der Mercaptogruppen ausgewählt ist aus

$-SCH_2CH_2COOH$, 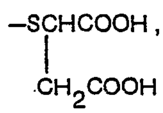, 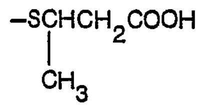, 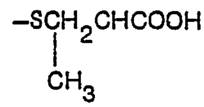,

$-SCH_2COOH$, $-SSO_3H$, $-SCH_2CH_2SO_3H$, 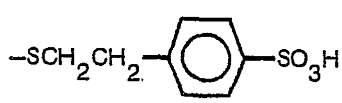,

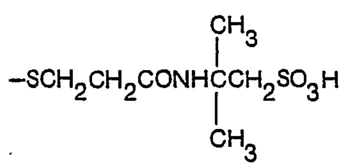 und $-SCH_2CH_2SO_2CH_2COOH$;

mit Ausnahme von Haarspülmitteln, die ein kationisches oberflächenaktives Mittel enthalten.

2. Verfahren nach Anspruch 1, umfassend eine Zugabe von mindestens einem Derivat von Keratinmaterial in einer Menge von 0,1 bis 5 Gew.% des Mittels.

3. Verfahren nach Anspruch 1, umfassend ferner eine Zugabe von mindestens einem oberflächenaktiven Mittel in einer Menge von 0,01 bis 5 Gew.%, ausgewählt aus anionischen oberflächenaktiven Mitteln, nicht ionischen oberflächenaktiven Mitteln und amphoteren oberflächenaktiven Mitteln.

4. Verfahren nach Anspruch 3, umfassend eine Zugabe von mindestens einem oberflächenaktiven Mittel in einer Menge von 1 bis 2 Gew.% des Haarspülmittels.

5. Verfahren nach Anspruch 3 oder 4, wobei mindestens eines der oberflächenaktiven Mittel ein ethoxyliertes Alkylsulfat mit einer linearen oder verzweigten Alkylgruppe 12 bis 16 Kohlenstoffatomen und mit 1 bis 4 Mol Ethylenoxid pro Molekül desselben ist, oder ein lineares oder verzweigtes Alkylsulfat mit durchschnittlich 12 bis 16 Kohlenstoffatomen.

6. Haarspülmittel, umfassend in flüssigem Medium 0,1 bis 10 Gew.% and mindestens eines Derivats von Keratinmaterial, ausgewählt aus Basensalzen von Produkten, die durch Oxidation von Keratinmaterial erhalten wurden, und Basensalzen von Derivaten der Mercaptogruppen von Produkten, die durch Reduktion von Keratinmaterial erhalten wurden, wobei das Derivat der Mercaptogruppe ausgewählt ist aus

$-SCH_2CH_2COOH$, 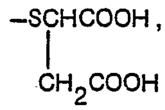, 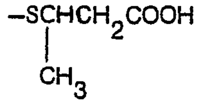, 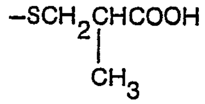,

$-SCH_2COOH$, $-SSO_3H$, $-SCH_2CH_2SO_3H$, 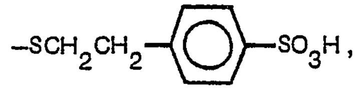,

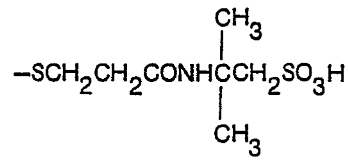 und $-SCH_2CH_2SO_2CH_2COOH$;

mit Ausnahme von Haarspülmitteln, welche ein kationisches oberflächenaktives Mittel enthalten.

7. Haarspülmittel nach Anspruch 6, umfassend mindestens ein Derivat von Keratinmaterial in einer Menge von 0,1 bis 5 Gew.% des Mittels.

8. Haarspülmittel nach Anspruch 6, ferner umfassend mindestens ein oberflächenaktives Mittel in einer Menge von 0,01 bis 5 Gew.%, ausgewählt aus anionischen oberflächenaktiven Mitteln, nicht ionischen oberflächenaktiven Mitteln und amphoteren oberflächenaktiven Mitteln.

9. Haarspülmittel nach Anspruch 8, wobei mindestens ein oberflächenaktives Mittel in einer Menge von 1 bis 2 Gew.% des Haarspülmittels enthalten sind.

10. Haarspülmittel nach Anspruch 8 oder 9, wobei mindestens eines der oberflächenaktiven Mittel ein ethoxyliertes Alkylsulfat mit einer linearen oder verzweigten Alkylgruppe mit 12 bis 16 Kohlenstoffatomen und mit 1 bis 4 Mol Ethylenoxyd pro Molekül desselben ist, oder ein lineares oder verzweigtes Alkylsulfat mit durchschnittlich 12 bis 16 Kohlenstoffatomen.

**Revendications pour les Etats contractants: BE CH DE FR IT LI NL**

1. Composition de rinçage pour les cheveux qui comprend, dans un milieu liquide, 0,1 à 10% en poids d'au moins un dérivé de matériau kératinique choisi parmi les sels avec des bases des produits obtenus par oxydation d'un matériau kératinique et les sels avec des bases de dérivés des groupes mercapto de produits obtenus par la réduction d'un matériau kératinique, où le dérivé du groupe mercapto est choisi parmi

$-SCH_2CH_2COOH$, 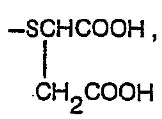, 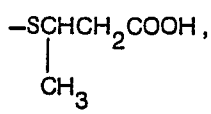, 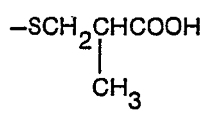,

$-SCH_2COOH$, $-SSO_3H$, $-SCH_2CH_2SO_3H$, 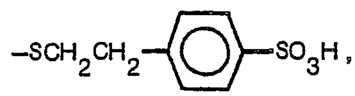,

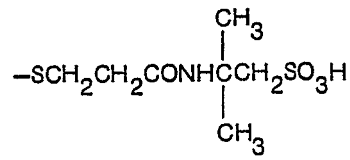 et $-SCH_2CH_2SO_2CH_2COOH$;

à l'exception des compositions de rinçage pour les cheveux contenant un agent tensio-actif cationique.

2. Composition de rinçage pour les cheveux selon la revendication 1, qui comprend 0,1—5% en poids de la composition, d'au moins un dérivé du matériau kératinique.

3. Composition de rinçage pour les cheveux selon la revendication 1, qui comprend en outre 0,01—5% en poids d'au moins un agent tensio-actif choisi parmi les agents tensio-actifs anioniques, les agents tensio-actifs non-ioniques et les agents tensio-actifs amphotères.

4. Composition de rinçage pour les cheveux selon la revendication 3, qui comprend 1—2% en poids de la composition, d'au moins un agent tensio-actif.

5. Composition de rinçage pour les cheveux selon la revendication 3 ou 4, dans laquelle au moins un des agents tensio-actifs est un sulfate d'alkyle éthoxylè possèdant un groupe alkyle linéaire ou ramifié ayant 12—16 atomes de carbone et contenant 1—4 moles d'oxyde d'éthylène par molécule de celui-ci ou un sulfate d'alkyle linéaire ou ramifié possédant une moyenne de 12—16 atomes de carbone.

**Revendications pour l'Etat contractant: AT**

1. Procédé de production d'une composition de rinçage pour les cheveux qui comprend l'addition à un milieu liquide, de 0,1 à 10% en poids d'au moins un dérivé d'un matériau kératinique choisi parmi les sels avec des bases des produits obtenus par oxydation d'un matériau kératinique et les sels avec des bases de dérivés des groupes mercapto de produits obtenus par réduction d'un matériau kératinique, où le dérivé du groupe mercapto est choisi parmi

$-SCH_2CH_2COOH$, 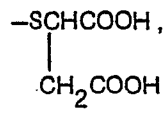, 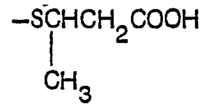, 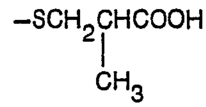,

$-SCH_2COOH$, $-SSO_3H$, $-SCH_2CH_2SO_3H$, 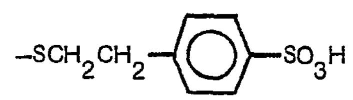,

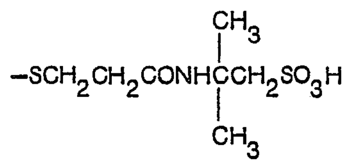 et $-SCH_2CH_2SO_2CH_2COOH$;

à l'exception des compositions de rinçage pour les cheveux contenant un agent tensio-actif cationique.

2. Procédé selon la revendication 1, qui comprend l'addition de 0,1 à 5% en poids de la composition, d'au moins un dérivé du matériau kératinique.

3. Procédé selon la revendication 1, qui comprend en outre l'addition de 0,01 à 5% en poids d'au moins un agent tensio-actif choisi parmi les agents tensio-actifs anioniques, les agents tensio-actifs non-ioniques et les agents tensio-actifs amphotères.

4. Procédé selon la revendication 3, qui comprend l'addition de 1 à 2% en poids de la composition, d'au moins un agent tensio-actif.

5. Procédé selon la revendication 3 ou 4, dans lequel au moins un des agents tensio-actifs est un sulfate d'alkyle éthoxylé possédant un groupe alkyle linéaire ou ramifié ayant 12 à 16 atomes de carbone et contenant 1 à 4 moles d'oxyde d'éthylène par molécule de celuici ou un sulfate d'alkyle linéaire ou ramifié possédant une moyenne de 12 à 16 atomes de carbone.

6. Composition de rinçage pour les cheveux, qui comprend, dans un milieu liquide, 0,1 à 10% en poids d'au moins un dérivé de matériau kératinique choisi parmi les sels avec des bases des produits obtenus par oxydation d'un matériau kératinique et les sels avec des bases des dérivés des groupes mercapto de produits obtenus par la réduction d'un matériau kératinique, où le dérivé du groupe mercapto est choisi parmi

$-SCH_2CH_2COOH$, 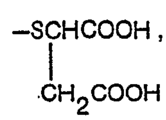, 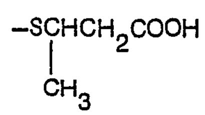, 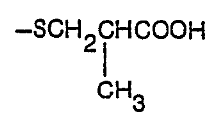,

$-SCH_2COOH$, $-SSO_3H$, $-SCH_2CH_2SO_3H$, 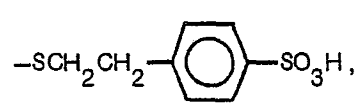,

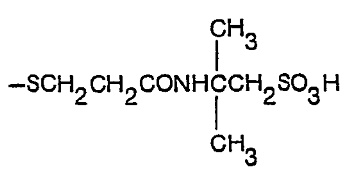 et $-SCH_2CH_2SO_2CH_2COOH$;

à l'exception des compositions de rinçage contenant un agent tensio-actif cationique.

7. Composition de rinçage pour les cheveux selon la revendication 6, qui comprend 0,1 à 5% en poids de la composition, d'au moins un dérivé du matériau kératinique.

8. Composition de rinçage pour les cheveux selon la revendication 6, qui comprend en outre 0,01 à 5% en poids d'au moins un agent tensio-actif choisi parmi les agents tensio-actifs anioniques, les agents

tensio-actifs non ioniques et les agents tensio-actifs amphotères.

9. Composition de rinçage pour les cheveux selon la revendication 8, qui comprend 1 à 2% en poids de la composition, d'au moins un agent tensio-actif.

10. Composition de rinçage pour les cheveux selon la revendication 8 ou 9, dans laquelle au moins un des agents tensio-actifs est un sulfate d'alkyle éthoxylé possèdant un groupe alkyle linéaire ou ramifié ayant 12 à 16 atomes de carbone et contenant 1 à 4 moles d'oxyde d'ethylène par molécule de celui-ci ou un sulfate d'alkyle linéaire ou ramifié possèdant une moyenne de 12 à 16 atomes de carbone.